# EUROPÄISCHE PATENTSCHRIFT

(11) **EP 2 839 012 B1**
(45) Veröffentlichungstag und Bekanntmachung des Hinweises auf die Patenterteilung: **28.08.2019**
(21) Anmeldenummer: 13717769.7
(22) Anmeldetag: 17.04.2013
(51) Int. Cl.: C12P 21/00, C12N 1/12, C12N 1/14

(54) **VERFAHREN ZUR ERHÖHUNG DER SEKRETION REKOMBINANTER PROTEINE**
METHOD FOR INCREASING THE SECRETION OF RECOMBINANT PROTEINS
PROCÉDÉ D'AUGMENTATION DE LA SÉCRÉTION D'UNE PROTÉINE RECOMBINANTE

(30) Priorität: 17.04.2012 EP 12164458
(43) Veröffentlichungstag der Anmeldung: 25.02.2015
(73) Patentinhaber: Greenovation Biotech GmbH, 79108 Freiburg (DE)
(72) Erfinder: JOST, Wolfgang, 79098 Freiburg (DE); KNAPPENBERGER, Mathias, 88487 Mietingen/Baltringen (DE); CLAUSSNITZER, Doreen, 80686 München (DE); SCHAAF, Andreas, 79104 Freiburg (DE)
(74) Vertreter: Sonn & Partner Patentanwälte
(86) Internationale Anmeldenummer: PCT/EP2013/057956
(87) Internationale Veröffentlichungsnummer: WO 2013/156504

(56) Entgegenhaltungen:
- US-A1- 2010 035 327
- DAVID F ET AL: "Optimization of antibody fragment production in Bacillus megaterium: the role of metal ions on protein secretion", JOURNAL OF BIOTECHNOLOGY,, Bd. 150, Nr. 1, 1. Oktober 2010 (2010-10-01), Seiten 115-124, XP009162656,
- BAUR ARMIN ET AL: "ENHANCED RECOVERY OF A SECRETED RECOMBINANT HUMAN GROWTH FACTOR USING STABILIZING ADDITIVES AND BY CO-EXPRESSION OF HUMAN SERUM ALBUMIN IN THE MOSS PHYSCOMITRELLA PATENS", PLANT BIOTECHNOLOGY JOURNAL, BLACKWELL, OXFORD, GB, Bd. 3, Nr. 3, 1. Mai 2005 (2005-05-01), Seiten 331-340, XP008073957, ISSN: 1467-7644, DOI: 10.1111/J.1467-7652.2005.00127.X
- BAUR A ET AL: "A fast and flexible PEG-mediated transient expression system in plants for high level expression of secreted recombinant proteins", JOURNAL OF BIOTECHNOLOGY, ELSEVIER SCIENCE PUBLISHERS, AMSTERDAM, NL, Bd. 119, Nr. 4, 10. Oktober 2005 (2005-10-10), Seiten 332-342, XP027663479, ISSN: 0168-1656 [gefunden am 2005-10-10]
- DECKER EVA L ET AL: "Current achievements in the production of complex biopharmaceuticals with moss bioreactors.", BIOPROCESS AND BIOSYSTEMS ENGINEERING JAN 2008 LNKD- PUBMED:17701058, Bd. 31, Nr. 1, Januar 2008 (2008-01), Seiten 3-9, XP019564175, ISSN: 1615-7591
- TWYMAN R M ET AL: "Molecular farming in plants: host systems and expression technology", TRENDS IN BIOTECHNOLOGY, ELSEVIER PUBLICATIONS, CAMBRIDGE, GB, Bd. 21, Nr. 12, 1. Dezember 2003 (2003-12-01), Seiten 570-578, XP004473523, ISSN: 0167-7799, DOI: 10.1016/J.TIBTECH.2003.10.002
- JIAN-DONG CUI ET AL: "Evaluation of Metal Ions and Surfactants Effect on Cell Growth and Exopolysaccharide Production in Two-Stage Submerged Culture of", APPLIED BIOCHEMISTRY AND BIOTECHNOLOGY ; PART A: ENZYME ENGINEERING AND BIOTECHNOLOGY, HUMANA PRESS INC, NEW YORK, vol. 168, no. 6, 7 April 2012 (2012-04-07) , pages 1394-1404, XP035141034, ISSN: 1559-0291, DOI: 10.1007/S12010-012-9865-7
- J. Matsunaga ET AL: "Osmolarity, a Key Environmental Signal Controlling Expression of Leptospiral Proteins LigA and LigB and the Extracellular Release of LigA", INFECTION AND IMMUNITY, vol. 73, no. 1, 1 January 2005 (2005-01-01), pages 70-78, XP055455833, ISSN: 0019-9567, DOI: 10.1128/IAI.73.1.70-78.2005
- THOMAS WUCHERPFENNIG ET AL: "Morphology engineering - Osmolality and its effect on Aspergillus niger morphology and productivity", MICROBIAL CELL FACTORIES,, vol. 10, no. 1, 29 July 2011 (2011-07-29), page 58, XP021109039, ISSN: 1475-2859, DOI: 10.1186/1475-2859-10-58
- Sadettin S. Ozturk ET AL: "Effect of medium osmolarity on hybridoma growth, metabolism, and antibody production", Biotechnology and Bioengineering, vol. 37, no. 10, 25 April 1991 (1991-04-25), pages 989-993, XP055018603, ISSN: 0006-3592, DOI: 10.1002/bit.260371015

## Beschreibung

Die vorliegende Erfindung betrifft die Optimierung von pflanzlichen oder pilzartigen rekombinanten Expressionssystemen durch den Einsatz geeigneter Expressionsverfahren.

Die Produktion von rekombinanten Proteinen im Konzentrationsbereich von ng/l bis µg/l in Pflanzen wurde in mehreren Publikationen beschrieben. Die Veröffentlichung WO 01/25456 A2 betrifft ein Verfahren zur Herstellung von Proteinen in Moos ohne Aufbrechen der Moosgewebe oder Mooszellen. Zudem wird genannt, dass die Verwendung von PVP im Kulturmedium die Produktivität erhöhen kann. Gezeigt wird dies am Beispiel der rekombinanten Produktion von VEGF₁₂₁, ein kleines glykosyliertes Homodimer von 28 kDa Größe (Baur et al., Journal of Biotechnology 119 (2005): 332-342). Baur et al. beschreibt die transiente Transformation von Moos mittels PEG. Das Medium zur Produktion von VEGF war frei von PVP oder PEG.

Drake et al., Plant Mol Biol 52 (2003): 233-241, beschreibt die rekombinante Expression von Antikörpern in einer Kultur von Tabakpflanzengewebe, nämlich Wurzeln. Die Antikörper wurden durch den Vorgang der Rhizosekretion im µg-Bereich sezerniert. Effektive pflanzliche Expressionssysteme, die auf der Rhizosekretion basieren, sind meist Haarwurzeln-Kulturen, welche durch *Agrobacterium rhizogenes*-Infektionen beliebiger Pflanzen stimuliert werden können (Gaume et al., Plant Cell Rep 21 (2003): 1188-1193).

Komarnysky et al., Plant Physiology 124 (2000): 927-933) beschreibt die Sekretion von rekombinanten Proteinen durch den Vorgang der Guttation, bei dem die Proteine mit der Guttationsflüssigkeit von Blättern abgesondert werden. Die täglichen Produktionsraten waren im Bereich von 1 µg/g Blattmasse.

Eine Tabak-Suspensionskultur zur rekombinanten Expression heterologer Proteine wird in Kwon et al. Biotechnology and Bioengineering 81(7) (2003): 870-875, beschrieben. Callus Zellen von transformierten Pflanzen wurden als Zelllinie zur Produktion von menschlichem IL-12 verwendet. Als Zusatz zum Zellkulturmedium wurden drei Polymere auf deren Eigenschaft Il-12 zu stabilisieren getestet. PVP und PEG hatten keinen Effekt. Gelatine führte zu einer Il-12-Stabilisierung bis ab Tag 5 der Kultur Proteasen im Medium zu einem Konzentrationsrückgang führten. Ähnliche Resultate werden in Lee et al., Journal of Biotechnology 96 (2002): 205-211 beschrieben.

LaCount et al., Biotechnology Letters 19(1) (1997): 93-96, beschreibt die Stabilisierung von rekombinant produzierten Antikörpern bzw. von GM-CSF durch Behandlung mit PVP zum Schutz vor Proteaseabbau.

Lee und Kim, Biotechnology Letters 24 (2002): 1779-1783, beschreiben die Verwendung von Pluronic F-68 und Polyethylenglycol zum Schutz von Zellen einer Tabaksuspensionskultur vor mechanischen Schäden in einem Rührtankbioreaktor.

Magnuson et al., Protein Expression and Purification 7 (1996): 220-228, beschreibt die Expression einer 50 kDa schweren Kette eines monoklonalen Antikörpers in einer Suspensionskultur von Tabakzellen. Durch Zugabe von PVP in das Kulturmedium wurde die Ausbeute um das 35-fache erhöht. Dies wurde auf eine Stabilisierung des Proteins sowie auf die Verhinderung der Aggregation und Anlagerung an Gefäßwänden zurückgeführt. 66% des Proteins wurden im Zytoplasma, 30% in der Membranfraktion und nur 4% im Medium gefunden. Große Proteine mit einer Molmasse größer als 50 kDa wurden als zu groß für Passagen durch die Zellwand angesehen, trotz sekretorischer Signalsequenzen, welche nur die Passage durch die Zellmembran signalisieren.

Schuster et al., Biotechnol. J. 2 (2002): 1-9, beschreibt die Produktion eines Antikörpers in Moos-Protoplasten. Die Ausbeute wurde von 0,1-0,5 µg/ml auf 8,2 µg/ml erhöht, durch Kultivierung in einer Mischung aus 3M- und W5-Medium mit vorhergehender effizienzgesteigerter Transformation nach Baur et al. (supra).

Tsoi und Doran, Biotechnol. Appl. Biochem. 35 (2002): 171-180, beschreiben verschiedene Medien zur Expression und deren Einfluss auf die Expression von Antikörpern durch Suspensionskulturen von Tabakzellen. Die Ausbeute der sekretierten Antikörper lag zwischen 20 und 200 µg / 50 ml Kulturmedium.

US 2010/035327 A1 betrifft ein universelles Kulturmedium-Additiv, welches aus Reis-Polysacchariden und Polypeptiden hergestellt wird. Es soll sowohl das Wachstum als auch die Sekretion von Zellprodukten erhöhen. Di Problematik der Sekretion durch eine Zellwand wird darin nicht angesprochen.

David et al., Journal of Biotechnology 150 (1) (2010): 115-124, betrifft die rekombinante Produktion von Antikörperfragmenten in *Bacillus megaterium,* also keiner Zelle mit Zellwand.

Baur et al., Plant Biotechnology Journal 3 (3) (2005): 331-340, beschreibt *P. patens* als Expressionssystem und eine erhöhte Sekretion von rekombinantem menschlichen Wachstumsfaktor (VEGF) durch die Verwendung von Additiven (PVP) und durch Expression von menschlichem Serumalbumin.

Decker et al., Bioprocess and Biosystems Engineering Jan 31 (1) (2008): 3-9, diskutiert allgemein Kulturbedingungen in Moos-Bioreaktoren und das Pflanzen-spezifische Glykoengineering.

Twyman et al., Trends in Biotechnology 21 (12) (2003): 570-578, erläutert die Verwendung von Pflanzen als rekombinante Expressionssysteme. Zum Thema der Ausbeute werden Transfektionssysteme, Signalsequenzen und mRNA-Stabilität diskutiert.

Trotz bekannter Verfahren zur Erhöhung der Produktivität von Pflanzen und Pflanzenzellen bei der rekombinanten Produktion heterologer Proteine sind pflanzliche Systeme noch immer weniger produktiv als tierische Zellen, wie beispielsweise etablierte CHO-Expressionssysteme. Es besteht daher ein fortwährender Bedarf die Produktivität von pflanzlichen Expressionssystemen weiter zu erhöhen. Zudem ist die Produktion von sekretierten Proteinen wünschenswert, sodass die produzierenden Zellen nicht zerstört werden müssen und weiterhin der Produktion dienen können.

Es ist daher ein Ziel der vorliegenden Erfindung verbesserte Expressionsverfahren und Mittel hierfür zur Verfügung zu stellen.

Die vorliegende Erfindung betrifft ein Verfahren zur Produktion eines rekombinanten Proteins in Zellen mit einer Zellwand, umfassend den Schritt der Sekretion des rekombinanten Proteins durch die Zellwand durch Expression des Proteins in den Zellen in einem Kulturmedium enthaltend eine Kombination von einem oberflächenaktiven Polymer und einwertigen Metallionen und mit einer Osmolarität von mindestens 0,22 osmol/L. Weitere Aspekte der vorliegenden Erfindung sind derartige Kulturmedien sowie Werkstoffzusammensetzungen. Der Schritt der Sekretion ist insbesondere eine Erhöhung der Sekretion. Die Erhöhung ist üblicherweise im Vergleich zu unbehandelten Zellen. Die Erfindung ist weiters wie in den Ansprüchen definiert. Im Folgenden werden besondere Ausführungen und vorzugsweise Parameter beschrieben, welche in Kombination miteinander vorgesehen werden können.

Für eine erfolgreiche und kostengünstige Produktion von Biopharmazeutika ist eine erfolgreiche Sekretion von Proteinen und eine gesteigerte Produktmenge [mg/l bis g/l] erforderlich. Zudem stellen Zellwände bedingt durch ihren (makro-)molekularen Aufbau, einen Größenausschlussfilter dar. Bei der Produktion von rekombinanten Proteinen in Pflanzen- oder Pilzzellen verhindert diese Tatsache eine Sekretion von Produkten größer als 90 kDa in das Kulturmedium, die für die weitere Prozessierung von Vorteil wäre. Vielmehr werden produzierte Proteine, sofern sie mit einem sekretorischen Signalpeptid versehen sind, zwar über die Zellmembran transportiert, akkumulieren dann aber im apoplastischen Raum, also dem Kompartiment zwischen Zellmembran und äußerem Abschluss der pflanzlichen Zellwand. Auch kleinere Proteine ab 50 kDa werden nur vermindert sekretiert. Die vorliegende Erfindung erhöht zunächst die Produktion von Proteinen und fördert zudem überraschender Weise die Sekretion dieser Proteine. Erfindungsgemäß produzierte Proteine können jeglicher Größe sein. Die besonderen Vorzüge stellen sich bei größeren Proteinen ein. Daher sind die erfindungsgemäß produzierten Proteine vorzugsweise größer als 40 kDa, größer als 50 kDa, größer als 55 kDa, größer als 60 kDa, größer als 65 kDa, größer als 70 kDa, größer als 75 kDa, größer als 80 kDa oder größer als 85 kDa, größer als 90 kDa. Die Proteine können auch Dimere oder Heteromere derartiger Proteine sein.

Die einwertigen Metallionen sind vorzugsweise ausgewählt aus Alkalimetallionen, wie Li, Na, K, Rb oder Cs. Insbesondere bevorzugt umfassen oder sind die einwertigen Metallionen Na-Ionen.

Bevorzugt liegt das Metallion, z.B. das Na-Ion, in einer Konzentration von mindestens 20 mM im Medium vor, insbesondere bevorzugt in einer Konzentration von mindestens 30 mM, mindestens 40 mM, mindestens 50 mM, mindestens 60 mM, mindestens 70 mM, mindestens 80 mM, mindestens 90 mM, oder mindestens 100 mM. Das Metallion in einer dieser Konzentrationen ist vorzugsweise Natrium.

Durch die Zugabe des Metallions wird die Osmolarität erhöht. Diese fördert die Sekretion durch die Zellwand, verursacht aber auch erheblichen Stress auf die Zelle, wodurch das Wachstum verlangsamt bzw. gänzlich gestoppt wird. Auch die Produktion von Protein wird verlangsamt. Vorzugsweise ist die Osmolarität mindestens 0,33 osmol/L, mindestens 0,34 osmol/L, mindestens 0,35 osmol/L, mindestens 0,36 osmol/L, mindestens 0,37 osmol/L, mindestens 0,38 osmol/L oder mindestens 0,4 osmol/L. Im speziellen ist die Osmolarität im Bereich von 0,32 osmol/L bis 0,6 osmol/L, oder auch von 0,35 osmol/L bis 0,55 osmol/L.

Das oberflächenaktive Polymer betrifft Substanzen, die die Oberflächenspannung einer Flüssigkeit oder die Grenzflächenspannung zwischen zwei Phasen herabsetzen und die Bildung von Dispersionen ermöglichen oder unterstützen bzw. als Lösungsvermittler wirken können. Überraschenderweise konnte sich durch die Verwendung eines derartigen Polymers zusammen mit einem Metallion bei hoher Osmolarität eine bedeutende Produktionssteigerung bei der Expression von sekretierten Proteinen in Zellen mit Zellwand einstellen. Das Polymer ist insbesondere ein nichtionisches wasserlösliches oberflächenaktives Polymer. Vorzugsweise ist es nicht denaturierend auf Proteine. Beispiele sind Polymere oder Copolymere ausgewählt aus Polyethern wie Polyalkylglykole, Polysorbate oder Polyvinylpyrrolidon, Polyvinylalkohol, wasserlösliche Cellulosederivate wie Hydroxypropylcellulose, Hydroxypropylmethylcellulose, Carboxymethylcellulose oder Hydroxyethylcellulose, VinylpyrrolidonVinylacetat-Copolymer (Copovidone), Polyvinylacetat, partiell hydrolysierter Polyvinylalkohol, Polyvinylalkohol-Polyethylenglykol-Copolymere und Mischungen daraus.

Vorzugsweise ist der Anteil an Carbonylsauerstoff (O=R₁) und/oder Ethersauerstoff (R₁-O-R₂) des Polymers zumindest 1 gew.-% des Molgewichts des Polymers. Ohne auf eine bestimmte Theorie beschränkt zu sein, scheint es, dass derartige Sauerstoffgruppen im Polymer zu einer Stabilisierung der Zelle führen, sodass das salzhaltige Medium mit hoher Osmolarität besser vertragen wird und dadurch eine hohe Produktivität ermöglicht wird. Vorzugsweise ist der Anteil an Carbonylsauerstoff und Ethersauerstoff des Polymers zumindest 1,5 gew.-%, zumindest 2,5 gew.-%, oder zumindest 5 gew.-%, speziell bevorzugt zumindest 10 gew.-%, zumindest 15 gew.-%, zumindest 20 gew.-%, zumindest 25 gew.-%. Der Anteil an Carbonylsauerstoff und Ethersauerstoff des Polymers ist vorzugsweise zwischen 1 gew.-% und 50 gew.-%, vorzugsweise zwischen 10 gew.-% und 42 gew.-%, insbesondre zwischen 5 gew.-% und 38 gew.-%, z.B. wie in Polyethylenglycol bei 36 gew.-% (nur Ethersauerstoff) oder wie in PVP bei 14,5 gew.-% (nur Carbonylsauerstoff).

Der Polyether ist vorzugsweise ein Polymer oder Copoylmer eines Polyalkylglycols, z.B. von Polyethylenglycol (PEG) oder von Polypropylenglycol oder Polyethylen-Polypropylenoxidcopolymere. Das polymerisierte Alkyl kann auch eine Mischung aus einzelnen Alkylen wie in einem Poloxamer sein. Vorzugsweise ist das Alkyl ausgewählt aus C₁-C₈-Akylen, insbesondere ausgewählt aus C₂-, C₃-, C₄-, C₅-, C₆-, C₇-Alkylen oder Mischungen hiervon. Ein Polyalkylglycol kann gemäß der Formel [-R-O-]ₙ aufgebaut sein, wobei R für die Alkylgruppe steht. Endständig können OH-Gruppen, Ester- oder Ethergruppen unabhängig voneinander vorgesehen werden. Die Ester- oder Ethergruppen können aus C₁-C₈-Gruppen wie bereits genannt oder aber auch aus längeren organischen Ketten, wie C₉-C₁₈ unabhängig voneinander ausgewählt sein. Sie können über eine Verbindungsgruppe, vorzugsweise eine C₁-C₁₂-Verbindungsgruppe, wie eine Arylgruppe (z.B. in Octoxinol (Triton X-100) oder Nonoxinol), verbunden sein. Vorzugsweise enthält der Polyether oder Polyalkylglycol mindestens eine OH-Gruppe, vorzugsweise mindestens 2 OH-Gruppen. Diese können endständig sein.

Das Molgewicht des oberflächenaktiven Polymers, z.B. PEG und dergleichen, ist vorzugsweise mindestens 500 Da, insbesondere bevorzugt mindestens 1.000 Da, mindestens 1.500 Da, mindestens 2.000 Da, mindestens 3.000 Da, mindestens 4.000 Da, mindestens 6.000 Da, mindestens 8.000 Da, mindestens 10.000 Da, mindestens 20.000 Da, mindestens 30.000 Da. Speziell bevorzugt ist das Molgewicht zwischen 500 Da und 2.000.000 Da, bevorzugt zwischen 1.000 Da und 200.000 Da oder zwischen 1.200 Da und 80.000 Da.

Das oberflächenaktive Polymer liegt vorzugsweise in einer Konzentration von mindestens 0,05 gew.-%, speziell bevorzugt von mindestens 0,08 %, mindestens 0,1 % oder mindestens 1,5 %, im Medium vor (alle %-Angaben in gew.-%).

Die Zellen werden in bestimmten Ausführungsformen in einer Suspensionskultur oder einer hydroponischen Kultur, insbesondere einer Haarwurzel-Kultur, kultiviert. Sekretierende Zellen werden mit dem erfindungsgemäßen Medium behandelt, um deren sekretorische Aktivität zu erhöhen.

Die Sekretion in das Medium erfolgt bei den erfindungsgemäßen Zellen meist aus dem apoplatischen Raum der Zelle. Für die Sekretion, ggf. in den apoplatischen Raum, wird das Protein in der Regel mit einer Signalsequenz für apoplatischen Raum bzw. für die Sekretion exprimiert. Geeignete Signalsequenzen sind hinreichend im Stand der Technik bekannt, z.B. in der einleitend erwähnten Literatur, und können erfindungsgemäß eingesetzt werden.

Das erfindungsgemäße Verfahren ist insbesondere geeignet, die Sekretion von Zellen mit einer Zellwand zu erhöhen. Derartige Zellen können aus einer pflanzlichen Zelle, einer Pilzzelle oder einer Algenzelle ausgewählt sein. "Zelle" wie hierin verwendet kann isolierte Zellen, vereinzelte Zellen oder eine Zelle in einem mehrzelligen Organismus betreffen. Eine pflanzliche Zelle kann eine pflanzliche Einzelzelle oder eine Zelle in einer Pflanze oder in einem Pflanzengewebe sein. In analoger Weise kann eine Pilzzelle eine Einzelzelle oder eine Zelle in einem Pilz oder einem Pilzgewebe sein. Die Algenzelle ist vorzugsweise eine Grünalgenzelle. Gewebe können z.B. ausgewählt sein aus Phloem, Xylem, Mesophyll, Stamm, Blätter, Thallus, Protonema, Chloronema, Caulonema, Rhizoiden oder Gametophoren. Die Zellen können Protoplasten oder Parenchymzellen, insbesondere Callus-Zellen, umfassen oder daraus bestehen.

Die im erfindungsgemäßen Verfahren einzusetzende Zelle ist vorzugsweise eine pflanzliche Zelle, vorzugsweise eines Mooses, insbesondere ausgewählt aus der Gruppe bestehend aus Laubmoosen und Lebermoosen, wobei Spezies aus den Gattungen Physcomitrella, Funaria, Sphagnum und Ceratodon, bzw. Marchantia und Sphaerocarpos besonders bevorzugt eingesetzt werden. Am meisten bevorzugt wird das erfindungsgemäße Verfahren unter Verwendung von Zellen, Pflanzen oder Pflanzengewebe wie Protonema des Laubmooses Physcomitrella patens durchgeführt. Weitere bevorzugte Pflanzen sind Tabak, Bohnen oder Linsen. Bevorzugt ist die Pflanze eine Wasserpflanze, z.B. der Gattungen Lemna, Spirodela, Landoltia, Wolffia oder Wolffiella.

Nach einer weiteren bevorzugten Ausführungsform der Erfindung wird die Zelle in einem Kulturmedium kultiviert, welches im Wesentlichen frei von Zuckern, Vitaminen und Phytohormonen bzw. funktionellen Fragmenten derselben ist. Geeignete Phytohormone sind beispielsweise Wachstumshormone, z.B. Auxine.

Vorzugsweise wird das erfindungsgemäße Verfahren unter photoautotrophen Wachstumsbedingungen durchgeführt. Das erfindungsgemäße Verfahren bietet die Möglichkeit der Kultivierung vollständiger ausdifferenzierter Pflanzen, Pflanzenzellen oder Algen unter standardisierbaren photoautotrophen Bedingungen, d.h. ohne das Erfordernis des Zusatzes von Zuckern, Vitaminen und Phytohormonen und dergleichen.

Im Grunde ist es natürliche möglich Zucker, Vitamine oder Phytohormone einzusetzen, insbesondere in Medien für Pilzzellen, die nicht photoautotroph wachsen können. Für Pilzzellen sollte eine katapole Kohlenstoffquelle vorgesehen werden. Aufgrund der autotrophen Eigenschaft der Zellen kann das Medium proteinfrei sein. Vorzugsweise ist das Medium ein sterilisiertes Medium, um die Infektionsgefahr zu verringern. Dem Medium kann auch ein Antibiotikum beigesetzt werden, um die Zellen vor Pathogenen, welche diese Zellen befallen, zu schützen.

Der pH-Wert des Kulturmediums ist vorzugsweise zwischen 3,5 und 8,5, insbesondere bevorzugt zwischen 4 und 8, oder zwischen 4,5 und 7, oder zwischen 5 und 6,5, insbesondere zwischen 5,5 und 6. Dadurch wird der pH-Wert auf optimale Wachstums oder Expressionsbedingungen optimiert.

Für optimales Wachstum oder erhöhte Proteinproduktion sollte das Medium Mikronährstoffe für die jeweiligen Zellen, insbesondere Mineralstoffe, enthalten. Vorzugsweise umfasst das Medium Nitrationen, vorzugsweise in einer Konzentration von mindestens 0,2 mM, Phosphationen, vorzugsweise in einer Konzentration von mindestens 0,05 mM, Sulfationen, vorzugsweise in einer Konzentration von mindestens 0,02 mM, Calciumionen, vorzugsweise in einer Konzentration von mindestens 0,1 mM, Kaliumionen, vorzugsweise in einer Konzentration von mindestens 0,1 mM, oder Kombinationen davon. Das Medium kann auch Natriumionen, vorzugsweise in einer Konzentration von mindestens 20 mM, enthalten.

In speziellen Ausführungsformen der vorliegenden Erfindung enthält das Verfahren noch einen weiteren Schritt vor dem genannten Schritt (der Erhöhung) der Sekretion des rekombinanten Proteins in dem genannten Medium. Mit diesem vorhergehenden Schritt werden die Zellen in einem Kulturmedium mit einer Osmolarität von weniger als 0,1 osmol/L wachsen gelassen, insbesondere ohne die genannte (Erhöhung der) Sekretion, wodurch Protein im Zellinneren oder im apoplatischen Raum der Zellen akkumuliert. Dieser vorhergehende Schritt fördert speziell die Proteinexpression, auch wenn diese Proteine unter diesen Bedingungen nicht erhöht sekretiert werden. Durch den folgenden Schritt (der Erhöhung) der Sekretion wie oben beschrieben können die vermehrt produzierten Proteine letztlich ohne Zerstörung der Zelle sekretiert und geerntet werden. Durch dieses 2-Schrittverfahren sind beträchtliche Produktionssteigerungen möglich.

Das Kulturmedium in diesem vorhergehenden Schritt kann im Grunde gleich zusammengesetzt sein wie das Kulturmedium im Sekretionsschritt - mit dem Unterschied der niedrigeren Osmolarität in Folge einer geringeren Metallion-Konzentration. Vorzugsweise enthält das Kulturmedium im vorhergehenden Schritt - analog oder unabhängig von der Zusammensetzung des Mediums im Sekretionsschritt - Nitrationen, vorzugsweise in einer Konzentration von mindestens 0,2 mM, Phosphationen, vorzugsweise in einer Konzentration von mindestens 0,05 mM, Sulfationen, vorzugsweise in einer Konzentration von mindestens 0,02 mM, Calciumionen, vorzugsweise in einer Konzentration von mindestens 0,1 mM, Kaliumionen, vorzugsweise in einer Konzentration von mindestens 0,1 mM, oder Kombinationen davon. Dadurch wird ein optimales Wachstum der Zellen ermöglicht. Vorzugsweise ist die Konzentration der Natriumionen maximal 20 mM, insbesondere bevorzugt maximal 15 mM oder maximal 10 mM.

Vorzugsweise ist das Kulturmedium im genannten vorhergehenden Schritt frei vom oberflächenaktiven Polymer oder enthält es in einer Konzentration von maximal 0,08 gew.-% oder maximal 0,01 gew.-% oder maximal 0,005 gew.-%.

Auch in diesem vorhergehenden Schritt kann das Kulturmedium im Wesentlichen frei von Zuckern, Vitaminen und Phytohormonen bzw. funktionellen Fragmenten derselben sein bzw. in alternativen Ausführungsformen kann es diese enthalten. Es kann steril sein oder ein Antibiotikum enthalten. Der pH-Wert des Kulturmediums im vorhergehenden Schritt ist vorzugsweise zwischen 3 und 8, insbesondere bevorzugt zwischen 3,5 und 7, oder zwischen 4 und 6,5, oder zwischen 4,1 und 6, insbesondere zwischen 4,2 und 5,5.

Vorzugsweise werden die Zellen im Kulturmedium des vorhergehenden Schrittes und/oder im Sekretionsschritt begast, insbesondere mit Luft oder Sauerstoff. Vorzugsweise enthält das Gas zur Begasung Kohlendioxid. Vorzugsweise ist der Anteil an Kohlendioxid im Gas zwischen 0 und 10 vol.-%, insbesondere bevorzugt zwischen 1 und 9 vol.-% oder zwischen 1,5 und 8 vol.-%, speziell bevorzugt zwischen 1,7 und 5 vol.-%. Kohlendioxid dient Pflanzen als Kohlenstoffquelle und sollte bei photoautotrophen Bedingungen vorgesehen werden.

Die Temperatur im vorhergehenden Schritt und/oder im Sekretionsschritt ist vorzugsweise zwischen 10 °C und 30 °C, speziell bevorzugt zwischen 15 °C und 26 °C.

Die Lichteinstrahlung gemessen an der Photonenflussdichte (in µE) ist vorzugsweise zwischen 1 und 3.000, insbesondere zwischen 100 und 2.200.

Der genannte vorhergehende Schritt kann für eine Zeit von 2 bis 30 Tage durchgeführt werden. In dieser Zeit erfolgt eine optimale Produktion des rekombinanten Proteins.

Der genannte Schritt (der Erhöhung) der Sekretion kann für eine Zeit von mindestens 2 Tage, vorzugsweise für 3 bis 120 Tage, durchgeführt werden. In dieser Zeit erfolgt eine optimale Sekretion des vorher produzierten oder in diesem Schritt produzierten rekombinanten Proteins.

Die 2-Stufenmethode beruht auf der semikontinuierlichen Kultivierung der Pflanzenzellkultur und besteht aus der Inokulation der Pflanzenzellen in ein Kulturmedium, der Inkubation bei spezifischen physikalischen Parametern sowie der Zugabe von einigen Medienkomponenten während des Prozesses. Während der Methode werden die Proteine zu einem definierten Zeitpunkt durch Wechsel der Mediumbedingungen aus dem apoplastischen Raum in den Kulturüberstand sekretiert.

Das erfindungsgemäße Verfahren beginnt üblicherweise - aber nicht notwendigerweise - mit der Inokulation der Zellen (z.B. 0,1 g/l Biotrockenmasse) in geeignete Bioreaktorsysteme (Wave-Reaktor, Chemostat, Röhrenreaktor, Schüttelkolben etc.) mit dem Medium sowie der Produktion von Biomasse bis zu einer definierten Zelldichte (z.B. 1-3 g/l). Durch Erhöhung der Osmolarität, z.B. durch Zugabe des Metallions und des Polymers (wobei das Polymer alleine die Osmolarität meist nicht erhöht und oft sogar verringert), wird die Sekretion initiiert. Über beide Phasen wird von den Zellen Protein produziert und exprimiert. Das erfindungsgemäße Verfahren ist beliebig skalierbar und in diversen Reaktoren und Mengen einsetzbar. Möglich sind Kleinstbioreaktoren (Mikrotiterplatte), Schüttelkolben, 5 L Chemostaten, 100 L Röhrenreaktor sowie Wavebioreaktoren (10 L bis 500 L).

Die Erfindung betrifft weiters ein Kulturmedium wie oben beschrieben, z.B. umfassend das oberflächenaktive Polymer in einer Menge von mindestens 0,05 gew.-% und einwertigen Metallionen in einer Konzentration von mindestens 20 mM und mit einer Osmolarität von mindestens 0,32 osmol/L. Das Kulturmedium ist für das oben beschriebene Verfahren geeignet und kann darin eingesetzt werden, insbesondere im Schritt der Sekretion(serhöhung).

Vorzugsweise umfasst das Kulturmedium Nitrationen, vorzugsweise in einer Konzentration von mindestens 0,2 mM, Phosphationen, vorzugsweise in einer Konzentration von mindestens 0,05 mM, Sulfationen, vorzugsweise in einer Konzentration von mindestens 0,02 mM, Calciumionen, vorzugsweise in einer Konzentration von mindestens 0,1 mM, Kaliumionen, vorzugsweise in einer Konzentration von mindestens 0,1 mM, oder Kombinationen davon. Vorzugsweise umfasst das Medium Natriumionen, vorzugsweise in einer Konzentration von mindestens 40 mM, oder mindestens 60 mM oder mindestens 80 mM.

Die Erfindung betrifft auch eine trockene Nährmediummischung zur Rekonstitution des Kulturmediums wie oben beschrieben. Die Nährmediummischung kann durch Zugabe von Wasser die obigen Mengen und Konzentrationen des genannten Kulturmediums ergeben. Somit betrifft die Erfindung auch ein Verfahren zur Herstellung des genannten Kulturmediums durch Lösen der genannten Inhaltsstoffe in einem wässrigen Lösungsmittel, vorzugsweise Wasser.

Die vorliegende Erfindung wird weiters durch die folgenden Figuren und Beispiele illustriert, ohne auf diese speziellen Ausführungsformen der Erfindung limitiert zu sein.

### Figuren:

Figur 1 zeigt die synergistische Wirkung einer durch NaCl erhöhten Osmolarität und des Polymers PEG auf die Sekretion eines rekombinant produzierten Antikörpers;
Figur 2 zeigt zusätzliche produktionssteigernde Effekte durch pflanzenwachstumsfördernde Mineralstoffe (CaNitrat);
Figur 3 zeigt die Wirkung unterschiedlicher Polymerkonzentrationen.

### Beispiele:

Die Erfindung liefert eine Methode, die die konstante Sekretion rekombinanter Proteine aus pflanzlichen Kulturen in das Kulturmedium ermöglicht. Hierbei werden die Produktionspflanzen submers in Flüssigkultur photoautotroph in definierten, mineralischen Medien kultiviert. Die Kulturmedien sind bezüglich der optimalen Nährstoffversorgung der Moospflanzen optimiert, bewirken an sich aber keine Abgabe größerer rekombinant produzierter Proteine ins Medium, die zunächst im Apoplasten akkumulieren und nach Aufschluss des Moosgewebes als "intrazellulär" messbar sind.

Durch Zugabe osmotisch aktiver Substanzen ("Sekretionskomponenten") wie NaCl und Polyethylenglycol (PEG) 4000 zum Kulturmedium kann diese apoplastische Rückhaltung der Proteine aufgehoben werden. Zu beobachten ist dann eine schnelle Abgabe der ackumulierten Proteine ins Medium und im weiteren Kulturverlauf eine konstante und zeitlich direkte Sekretion neu synthetisierter Proteine.

### Versuchsbeispiel: Vergleich der Proteinsekretion in Erlenmeyer-Kulturen

500 ml Erlenmeyer-Kolben wurden mit 180 ml sterilem Knop- oder WM01-Medium befüllt, auf pH 4,5-6 eingestellt (2-(N-Morpholino)ethansulfonsäure-Puffer, "MES") und mit frisch turraxierter Physcomitrella-Protonema Supensionskultur eines rekombinanten, Testprotein-produzierenden Stammes beimpft. Die Inokulationsdichte betrug 0,1 g Trockengewicht/l. Die Kulturen wurden steril und gasdurchlässig verschlossen und mit 2% CO₂ angereicherter Atmosphäre kultiviert. Nach Wachstum auf eine Zelldichte von 1-3 g/l erfolgte die Zugabe der Sekretionskomponenten in Form eines sterilen Konzentrats. Während der Kultivierung wurden die Parameter Kulturdichte (g Trockengewicht/l), intrazellulärer und extrazellulärer IgG-Titer (mg/l) regelmäßig bestimmt.

### Ergebnisse:

Die folgenden Testproteine wurden produziert:

**Tabelle 1: Sezernierte Testproteine**

| Protein | MW [kDa] | Sekretion ohne Sekretions-Medium | Sekretion mit Sekretions-Medium (NaCl, PEG) |
|---|---|---|---|
| IgG | 145 | Nein | Ja |
| alpha Galaktosidase | 80 | Nein | Ja |
| VEGF₁₂₁ | 50 | Ja | Ja |
| Epo | 30 | Ja | Ja |
| HSA | 67 | Ja | Ja |

**Tabelle 2: Medien (alle Konzentrations-Angaben in mg/l, * außer für MES):**

| | 1 | 2 | 3 | 4 | 5 | 6 |
|---|---|---|---|---|---|---|
| Nährmedien | KNOP | 1/10 KNOP | BM | WM01 | Murashige & Skoog | Linsmaier & Skoog |
| KH₂PO₄ | 250 | 25 | 250 | 500 | 170 | 170 |
| NH₄NO₃ | | | | | 1650 | 1650 |
| KNO₃ | | | | | 1900 | 1900 |
| KCl | 250 | 25 | 250 | 500 | | |
| MgSO₄ x 7 H₂O | 250 | 25 | 250 | 500 | 180,7 | |
| Ca (NO₃) 2 x 4 H₂O | 1000 | 100 | 1000 | 2000 | | |
| CaCl | | | | | 332,2 | 332,2 |
| NaCl | | | | | | |
| MES* | | | 5 mM | 5 mM | | |

Zusätzlich können Mikroelemente wie H₃BO₃, FeSO₄, Fe-NaEDTA, CoCl₂, CuSO₄, KI, MnCl₂, MnSO₄, Na₂MoO₄, NiCl₂, Na₂SeO₃, Zn-Acetat, oder Vitamine wie Folsäure, myo-Inositol, Nicotinsäure Thiamin-HCl, Pyridoxin-HCl, Biotin oder auch Glycin zugefügt werden. Für Pilzkulturen wird zusätzlich eine katabole Kohlenhydratquelle wie Glucose, Mannose oder Mannitol zugesetzt.

### Beispiel 1: Produktionssteigerung durch PEG und erhöhte Osmolarität

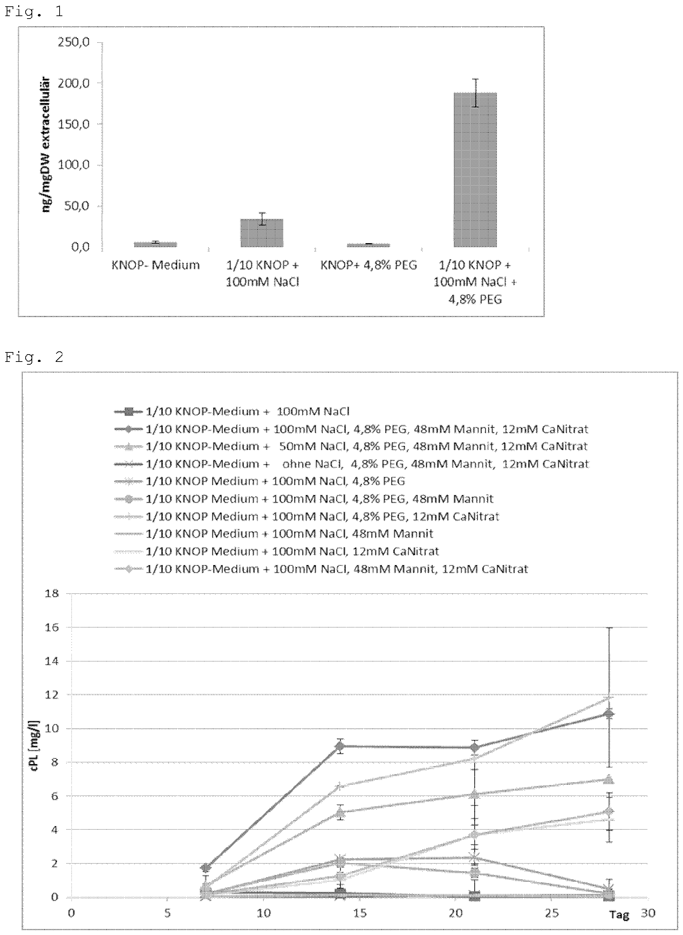

Ein IgG Antikörper wurde wie beschrieben bei folgenden Parametern in P. patents exprimiert: Lichtrhythmus [h] 16/8, Ausgangsmedium KNOP, Temperatur 22 °C, Kulturgefäß: Kolben; Medium "1/10 KNOP NaCl" enthält zusätzlich zu Medium "1/10 KNOP" 100mM NaCl. Medium "KNOP PEG" bzw. "1/10 KNOP NaCl PEG" enthält zusätzlich zu Medium "KNOP" bzw. "1/10 KNOP NaCl" 4,8 gew.-% PEG-4000. Ergebnisse der Antikörper-Produktion nach 6 Wochen sind in Tabelle 3 und in Fig. 1 dargestellt.

**Tabelle 3: PEG und NaCl (100mM zur Osmolaritätssteigerung)**

| | **intrazellulär** | **extrazellulär** | **extra / total** |
|---|---|---|---|
| **Kolben** | **ng IgG / mg Trockengewicht** | **ng IgG / mg Trockengewicht** | **%** |
| Knop, Versuch 1 | 62,3 | 4,8 | **7%** |
| Knop, Versuch 2 | 88,5 | 6,6 | **7%** |
| Knop, Versuch 3 | 84,1 | 6,4 | **7%** |
| **Durchschnitt** | **78,3** | **5,9** | **7%** |
| 1/10 Knop NaCl, Versuch 1 | 27,3 | 29,7 | **52%** |
| 1/10 Knop NaCl, Versuch 2 | 24,0 | 30,8 | **56%** |
| 1/10 Knop NaCl, Versuch 3 | 19,5 | 43,1 | **69%** |
| **Durchschnitt** | **23,6** | **34,5** | **59%** |
| Knop PEG, Versuch 1 | 35,9 | 4,3 | **11%** |
| Knop PEG, Versuch 2 | 25,0 | 4,5 | **15%** |
| Knop PEG, Versuch 3 | 33,1 | 4,5 | **12%** |
| **Durchschnitt** | **31,4** | **4,4** | **13%** |
| 1/10 Knop NaCl PEG, Versuch 1 | 27,7 | 180,7 | **87%** |
| 1/10 Knop NaCl PEG, Versuch 2 | 30,1 | 175,3 | **85%** |
| 1/10 Knop NaCl PEG, Versuch 3 | 36,2 | 208,2 | **85%** |
| **Durchschnitt** | **31,3** | **188,1** | **86%** |

Durch die Kombination von NaCl und PEG wurde eine Produktivitätssteigerung und Sekretionssteigerung beobachtet, welche weit über die Effekte von NaCl alleine und PEG alleine hinausgehen. Ein synergistischer Effekt liegt eindeutig vor.

### Beispiel 2: Produktionssteigerung durch PEG und erhöhte Osmolarität und Nitrat

Eine lambda-Kette eines IgG1 Antikörpers wurde wie beschrieben bei folgenden Parametern in *P. patents* exprimiert: Lichtrhythmus [h] 16/8, Ausgangsmedium KNOP, Temperatur 25 °C, Lichtintenisität 40µE/m²s, Kulturgefäß: Kolben; Ergebnisse der Antikörper-Produktion nach verschiedenen Zeitintervallen sind in Tabelle 4 und in Fig. 2 dargestellt.

**Tabelle 4: PEG, NaCl (Osmolarität) und Nitrat (Konzentration IgG1 in µg/ml)**

| Tag der Kultivierung | 7 | 14 | 21 | 28 |
|---|---|---|---|---|
| 1/10 KNOP-Medium + 100mM NaCl | 0,279 | 0,239 | 0,053 | 0,059 |
| 1/10 KNOP-Medium + 100mM NaCl, 4,8% PEG, 48mM Mannit, 12mM CaNitrat | 1,719 | 8,947 | 8,882 | 10,872 |
| 1/10 KNOP-Medium + 50mM NaCl, 4,8% PEG, 48mM Mannit, 12mM CaNitrat | 0,717 | 5,04 | 6,125 | 7,001 |
| 1/10 KNOP-Medium + ohne NaCl, 4,8% PEG, 48mM Mannit, 12mM CaNitrat | 0,048 | 0,086 | 0,123 | 0,122 |
| 1/10 KNOP Medium + 100mM NaCl, 4,8% PEG | 0,19 | 2,248 | 2,363 | 0,494 |
| 1/10 KNOP Medium + 100mM NaCl, 4,8% PEG, 48mM Mannit | 0,214 | 2,041 | 1,453 | 0,213 |
| 1/10 KNOP Medium + 100mM NaCl, 4,8% PEG, 12mM CaNitrat | 0,578 | 6,574 | 8,226 | 11,83 |
| 1/10 KNOP Medium + 100mM NaCl, 48mM Mannit | 0,043 | 0,132 | 0,12 | 0,088 |
| 1/10 KNOP Medium + 100mM NaCl, 12mM CaNitrat | 0,159 | 1,027 | 3,715 | 4,606 |
| 1/10 KNOP-Medium + 100mM NaCl, 48mM Mannit, 12mM CaNitrat | 0,185 | 1,259 | 3,704 | 5,087 |

Nach diesen Ergebnissen bewirkt Mannit keinen Einfluss auf das Sekretionsverhalten. Nitrat ist aufgrund des verbesserten Pflanzenwachstums förderlich. Ebenso zeigten sich in weiteren Versuchen positive Effekte von Kalium-, Phosphat-, Sulfat-, oder Calcium-Ionen, im Bereich von 1 bis 10 mM, die auf verbessertes Pflanzenwachstum zurückzuführen sind. In diesen niedrigen Konzentrationen, ohne die Osmolarität wesentlich zu erhöhen, wurde kein Effekt auf die Sekretion festgestellt.

### Beispiel 3: Unterschiedliche Polymere und Polymerkonzentrarionen

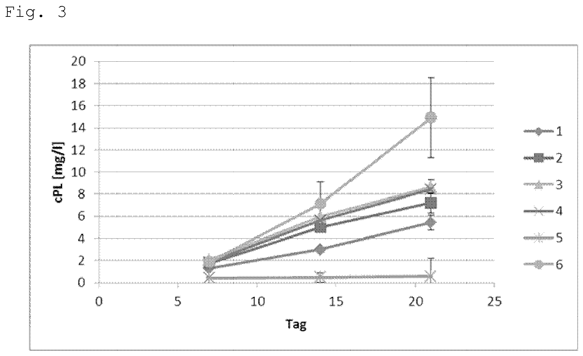

Eine lambda-Kette eines IgG1 Antikörpers wurde wie beschrieben bei folgenden Parametern in *P. patents* exprimiert: Lichtrhythmus [h] 16/8, Ausgangsmedium KNOP/BM, Temperatur 25 °C, Lichtintenisität 40µE/m²s, Kulturgefäß: Kolben; Ergebnisse der Antikörper Produktion nach verschiedenen Zeitintervallen sind in Tabellen 5 und 6 und in Fig. 3 dargestellt.

**Tabelle 5: Mediumzusammensetzung und IgG-Produktion (Konzentrationen in mM)**

| **Medium** | 1 | 2 | 3 | 4 | 5 | 6 |
|---|---|---|---|---|---|---|
| Magnesium-Ionen | 0,1014 | 0,501 | 0,501 | 0,501 | 0,501 | 0,501 |
| Calcium-Ionen | 12,4235 | 14,09 | 14,09 | 14,09 | 14,09 | 14,09 |
| Chlorid-Ionen | 100 | 100 | 100 | 100 | 100 | 100 |
| Sulfat-Ionen | 0,1059 | 0,501 | 0,501 | 0,501 | 0,501 | 0,501 |
| Nitrat- Ionen | 24,847 | 28,185 | 28,185 | 28,185 | 28,185 | 28,185 |
| Kalium-Ionen | 0,1837 | 0,9075 | 0,9075 | 0,9075 | 0,9075 | 0,9075 |
| Natrium-Ionen | 100 | 100 | 100 | 100 | 100 | 100 |
| Phosphat-Ionen | 0,1837 | 0,9075 | 0,9075 | 0,9075 | 0,9075 | 0,9075 |
| MES | 0 | 2,4705 | 2,4705 | 2,4705 | 2,4705 | 2,4705 |
| Mannit | 48 | 48 | 48 | 48 | 48 | 48 |
| PEG [%] w/V | 4,8 | 4,8 | 1 | 0,5 | | |
| pH | 5,8 | 5,9 | 5,9 | 5,9 | 5,9 | 5,9 |
| Tween 20 [w/v] | | | | | 0,05 | 0,01 |

**Tabelle 6: Ergebnisse der IgG-Produktion in µg/ml**

| Medium Nr. | 7 d | 14 d | 21 d |
|---|---|---|---|
| 1 | 1,26 | 2,98 | 5,43 |
| 2 | 1,75 | 5,01 | 7,2 |
| 3 | 2,05 | 5,95 | 8,66 |
| 4 | 1,85 | 5,61 | 8,42 |
| 5 | 0,41 | 0,46 | 0,57 |
| 6 | 1,88 | 7,13 | 14,9 |

Basierende auf diesen Ergebnissen lässt sich festhalten, dass niedrige Konzentrationen von PEG keinen negativen Einfluss auf die Sekretion haben, solange PEG vorhanden ist. Tween ist in niedrigeren Konzentrationen (Medium 6) wirksamer als bei hohen Konzentrationen (Medium 5).

## Patentansprüche

1. Verfahren zur Produktion eines rekombinanten Proteins in pflanzlichen Zellen mit einer Zellwand, umfassend den Schritt der Sekretion des rekombinanten Proteins durch die Zellwand durch Expression des Proteins in den pflanzlichen Zellen in einem Kulturmedium enthaltend eine Kombination von einem oberflächenaktiven Polymer und einwertigen Metallionen und mit einer Osmolarität von mindestens 0,32 osmol/L.

2. Verfahren nach Anspruch 1, **dadurch gekennzeichnet, dass** die Sekretion in das Medium aus dem apoplatischen Raum der Zelle erfolgt.

3. Verfahren nach Anspruch 1 oder 2, **dadurch gekennzeichnet, dass** die pflanzliche Zelle eine Zelle in einer Pflanze oder in einem Pflanzengewebe ist, speziell bevorzugt eine Mooszelle ist.

4. Verfahren nach einem der Ansprüche 1 bis 3, **dadurch gekennzeichnet, dass** die Zelle in einer Suspensionskultur kultiviert wird.

5. Verfahren nach einem der Ansprüche 1 bis 4, **dadurch gekennzeichnet, dass** das Metallion ein Alkalimetallion, vorzugsweise Natrium, ist, und/oder das Metallion in einer Konzentration von mindestens 20 mM im Medium vorliegt.

6. Verfahren nach einem der Ansprüche 1 bis 5, **dadurch gekennzeichnet, dass** das oberflächenaktive Polymer ein Polyalkylglycol, vorzugsweise Polyethylenglycol ist, und/oder das oberflächenaktive Polymer in einer Konzentration von mindestens 0,05 gew.-% im Medium vorliegt.

7. Verfahren nach einem der Ansprüche 1 bis 6, **dadurch gekennzeichnet, dass** das Medium Nitrationen, vorzugsweise in einer Konzentration von mindestens 0,2 mM, Phosphationen, vorzugsweise in einer Konzentration von mindestens 0,05 mM, Sulfationen, vorzugsweise in einer Konzentration von mindestens 0,02 mM, Calciumionen, vorzugsweise in einer Konzentration von mindestens 0,1 mM, Kaliumionen, vorzugsweise in einer Konzentration von mindestens 0,1 mM, Natriumionen, vorzugsweise in einer Konzentration von mindestens 20 mM, oder Kombinationen davon, umfasst.

8. Verfahren nach einem der Ansprüche 1 bis 7, **dadurch gekennzeichnet, dass** vor dem genannten Schritt der Sekretion des rekombinanten Proteins in dem genannten Medium die Zellen in einem vorhergehenden Schritt in einem Kulturmedium mit einer Osmolarität von weniger als 0,1 osmol/L wachsen gelassen werden, insbesondere ohne die genannte Sekretion, wodurch Protein im Zellinneren oder im apoplatischen Raum der Zellen akkumuliert.

9. Verfahren nach Anspruch 8, **dadurch gekennzeichnet, dass** das Kulturmedium im genannten vorhergehenden Schritt Nitrationen, vorzugsweise in einer Konzentration von mindestens 0,2 mM, Phosphationen, vorzugsweise in einer Konzentration von mindestens 0,05 mM, Sulfationen, vorzugsweise in einer Konzentration von mindestens 0,02 mM, Calciumionen, vorzugsweise in einer Konzentration von mindestens 0,1 mM, Kaliumionen, vorzugsweise in einer Konzentration von mindestens 0,1 mM, oder Kombinationen davon, umfasst; und/oder Natriumionen in einer Konzentration von maximal 20 mM umfasst.

10. Verfahren nach Anspruch 8 oder 9, **dadurch gekennzeichnet, dass** das Kulturmedium im genannten vorhergehenden Schritt das oberflächenaktive Polymer nicht oder in einer Konzentration von maximal 0,08 gew.-% umfasst.

11. Verfahren nach einem der Ansprüche 8 bis 10, **dadurch gekennzeichnet, dass** der genannte vorhergehende Schritt für eine Zeit von 2 bis 30 Tage durchgeführt wird.

12. Verfahren nach einem der Ansprüche 1 bis 11, **dadurch gekennzeichnet, dass** der genannte Schritt der Sekretion für eine Zeit von mindestens 2 Tage, vorzugsweise für 3 bis 120 Tage, durchgeführt wird.

13. Kulturmedium geeignet zur Sekretionsförderung nach einem Verfahren nach einem der Ansprüche 1 bis 12 umfassend das oberflächenaktive Polymer in einer Menge von mindestens 0,05 gew.-% und einwertige Metallionen in einer Konzentration von mindestens 20 mM und mit einer Osmolarität von mindestens 0,32 osmol/L.

14. Kulturmedium nach Anspruch 13 umfassend Nitrationen, vorzugsweise in einer Konzentration von mindestens 0,2 mM, Phosphationen, vorzugsweise in einer Konzentration von mindestens 0,05 mM, Sulfationen, vorzugsweise in einer Konzentration von mindestens 0,02 mM, Calciumionen, vorzugsweise in einer Konzentration von mindestens 0,1 mM, Kaliumionen, vorzugsweise in einer Konzentration von mindestens 0,1 mM, Natriumionen, vorzugsweise in einer Konzentration von mindestens 40 mM, oder Kombinationen davon.

15. Trockene Nährmediummischung zur Rekonstitution des Kulturmediums nach Anspruch 13 oder 14.

## Claims

1. Method for producing a recombinant protein in plant cells having a cell wall, comprising the step of secreting the recombinant protein through the cell wall by expression of the protein in the plant cells in a culture medium containing a combination of a surface-active polymer and monovalent metal ions and having an osmolarity of at least 0.32 osmol/L.

2. Method according to claim 1, **characterised in that** the secretion into the medium occurs from the apoplastic space of the cell.

3. Method according to either claim 1 or claim 2, **characterised in that** the plant cell is a cell in a plant or plant tissue, a moss cell being particularly preferred.

4. Method according to any of claims 1 to 3, **characterised in that** the cell is cultured in a suspension culture.

5. Method according to any of claims 1 to 4, **characterised in that** the metal ion is an alkali metal ion, preferably sodium, and/or the metal ion is present in the medium in a concentration of at least 20 mM.

6. Method according to any of claims 1 to 5, **characterised in that** the surface-active polymer is a polyalkyl glycol, preferably polyethylene glycol, and/or the surface-active polymer is present in the medium in a concentration of at least 0.05 wt.%.

7. Method according to any of claims 1 to 6, **characterised in that** the medium comprises nitrate ions, preferably in a concentration of at least 0.2 mM, phosphate ions, preferably in a concentration of at least 0.05 mM, sulfate ions, preferably in a concentration of at least 0.02 mM, calcium ions, preferably in a concentration of at least 0.1 mM, potassium ions, preferably in a concentration of at least 0.1 mM, sodium ions, preferably in a concentration of at least 20 mM, or combinations thereof.

8. Method according to any of claims 1 to 7, **characterised in that**, prior to said step of secretion of the recombinant protein in said medium, the cells are grown in a preceding step in a culture medium having an osmolarity of less than 0.1 osmol/L, in particular without said secretion, causing protein to accumulate within the cell or in the apoplastic space of the cells.

9. Method according to claim 8, **characterised in that** the culture medium, in said preceding step, comprises nitrate ions, preferably in a concentration of at least 0.2 mM, phosphate ions, preferably in a concentration of at least 0.05 mM, sulfate ions, preferably in a concentration of at least 0.02 mM, calcium ions, preferably in a concentration of at least 0.1 mM, potassium ions, preferably in a concentration of at least 0.1 mM, or combinations thereof; and/or comprises sodium ions in a concentration of at most 20 mM.

10. Method according to either claim 8 or claim 9, **characterised in that** the culture medium, in said preceding step, does not comprise the surface-active polymer or comprises it in a concentration of at most 0.08 wt.%.

11. Method according to any of claims 8 to 10, **characterised in that** said preceding step is carried out for a period of from 2 to 30 days.

12. Method according to any of claims 1 to 11, **characterised in that** said secretion step is carried out for a period of at least 2 days, preferably for 3 to 120 days.

13. Culture medium suitable for promoting secretion by means of a method according to any of claims 1 to 12, comprising the surface-active polymer in an amount of at least 0.05 wt.% and monovalent metal ions in a concentration of at least 20 mM and having an osmolarity of at least 0.32 osmol/L.

14. Culture medium according to claim 13 comprising nitrate ions, preferably in a concentration of at least 0.2 mM, phosphate ions, preferably in a concentration of at least 0.05 mM, sulfate ions, preferably in a concentration of at least 0.02 mM, calcium ions, preferably in a concentration of at least 0.1 mM, potassium ions, preferably in a concentration of at least 0.1 mM, sodium ions, preferably in a concentration of at least 40 mM, or combinations thereof.

15. Dry growth medium mixture for reconstituting the culture medium according to either claim 13 or claim 14.

## Revendications

1. Procédé de production d'une protéine recombinante dans des cellules végétales avec une paroi de cellule, comprenant l'étape de sécrétion de la protéine recombinante par la paroi de cellule par expression de la protéine dans les cellules végétales dans un milieu de culture contenant une combinaison d'un polymère tensioactif et d'ions métalliques monovalents et avec une osmolarité d'au moins 0,32 osmol/L.

2. Procédé selon la revendication 1, **caractérisé en ce que** la sécrétion a lieu dans le milieu à partir de l'espace apoplastique de la cellule.

3. Procédé selon la revendication 1 ou 2, **caractérisé en ce que** la cellule végétale est une cellule dans une plante ou dans un tissu végétal, spécialement de préférence est une cellule de mousse.

4. Procédé selon l'une quelconque des revendications 1 à 3, **caractérisé en ce que** la cellule est cultivée dans une culture de suspension.

5. Procédé selon l'une quelconque des revendications 1 à 4, **caractérisé en ce que** l'ion métallique est un ion métallique alcalin, de préférence du sodium, et/ou l'ion métallique se présente dans une concentration d'au moins 20 mM dans le milieu.

6. Procédé selon l'une quelconque des revendications 1 à 5, **caractérisé en ce que** le polymère tensioactif est un polyalkylglycole, de préférence polyéthylèneglycole, et/ou le polymère tensioactif se présente dans une concentration d'au moins 0,05 % en poids dans le milieu.

7. Procédé selon l'une quelconque des revendications 1 à 6, **caractérisé en ce que** le milieu comporte des ions de nitrate, de préférence dans une concentration d'au moins 0,2 mM, des ions de phosphate, de préférence dans une concentration d'au moins 0,05 mM, des ions de sulfate, de préférence dans une concentration d'au moins 0,02 mM, des ions de calcium, de préférence dans une concentration d'au moins 0,1 mM, des ions de potassium, de préférence dans une concentration d'au moins 0,1 mM, des ions de sodium, de préférence dans une concentration d'au moins 20 mM, ou des combinaisons de ceux-ci.

8. Procédé selon l'une quelconque des revendications 1 à 7, **caractérisé en ce qu'**avant l'étape citée de la sécrétion de la protéine recombinante dans le milieu cité, les cellules dans une étape sont laissées à croître précédente dans un milieu de culture avec une osmolarité de moins de 0,1 osmol/L, en particulier sans la sécrétion citée, moyennant quoi la protéine s'accumule à l'intérieur de la cellule ou dans l'espace apoplastique des cellules.

9. Procédé selon la revendication 8, **caractérisé en ce que** le milieu de culture dans l'étape précédente citée comporte des ions de nitrate, de préférence dans une concentration d'au moins 0,2 mM, des ions de phosphate, de préférence dans une concentration d'au moins 0,05 mM, des ions de sulfate, de préférence dans une concentration d'au moins 0,02 mM, des ions de calcium, de préférence dans une concentration d'au moins 0,1 mM, des ions de potassium, de préférence dans une concentration d'au moins 0,1 mM, ou des combinaisons de ceux-ci ; et/ou des ions de sodium dans une concentration de 20 mM au maximum.

10. Procédé selon la revendication 8 ou 9, **caractérisé en ce que** le milieu de culture ne comporte pas dans l'étape précédente citée le polymère tensioactif ou dans une concentration de 0,08 % en poids au maximum.

11. Procédé selon l'une quelconque des revendications 8 à 10, **caractérisé en ce que** l'étape précédente citée est réalisée pendant une durée de 2 à 30 jours.

12. Procédé selon l'une quelconque des revendications 1 à 11, **caractérisé en ce que** l'étape citée de sécrétion est réalisée pendant une durée d'au moins 2 jours, de préférence de 3 à 120 jours.

13. Milieu de culture approprié pour le transport de sécrétion selon un procédé selon l'une quelconque des revendications 1 à 12 comprenant le polymère tensioactif dans une quantité d'au moins 0,05 % en poids et des ions métalliques monovalents dans une concentration d'au moins 20 mM et avec une osmolarité d'au moins 0,32 osmol/L.

14. Milieu de culture selon la revendication 13 comprenant des ions de nitrate, de préférence dans une concentration d'au moins 0,2 mM, des ions de phosphate, de préférence dans une concentration d'au moins 0,05 mM, des ions de sulfate, de préférence dans une concentration d'au moins 0,02 mM, des ions de calcium, de préférence dans une concentration d'au moins 0,1 mM, des ions de potassium, de préférence dans une concentration d'au moins 0,1 mM, et/ou des ions de sodium, de préférence dans une concentration de 40 mM au maximum, ou des combinaisons de ceux-ci.

15. Mélange de milieu nutritif sec pour la reconstitution du milieu de culture selon la revendication 13 ou 14.
